# EUROPEAN PATENT SPECIFICATION

(11) **EP 2 433 626 B1**
(45) Date of publication and mention of the grant of the patent: **04.11.2015**
(21) Application number: 11187136.4
(22) Date of filing: 28.09.2004
(51) Int. Cl.: A61K 31/13, A61K 31/136

(54) **Compositions and methods for treatment of cardiovascular disorders and diseases**
Zusammensetzungen und Verfahren zur Behandlung kardiovaskulärer Erkrankungen
Compositions et procédés pour le traitement des troubles cardiovasculaires et maladies

(30) Priority: 25.11.2003 US 524616 P; 13.05.2004 US 570496 P
(43) Date of publication of application: 28.03.2012
(62) Divisional of application: 04770579.3
(73) Proprietor: Technion Research & Development Foundation Ltd., 32000 Haifa (IL); Rappaport Family Institute for Research in the Medical Sciences, 31096 Haifa (IL)
(72) Inventor: Youdim, Moussa B.H., 32763 Haifa (IL); Binah, Ofer, 36001 Nofit (IL); Abassi, Zaid A., 35156 Haifa (IL); Barac, Yaron, 26364 Kiryat Motzkin (IL)
(74) Representative: Vossius & Partner Patentanwälte Rechtsanwälte mbB

(56) References cited:
- WO-A-99/37293
- ELIASH S ET AL: "Rasagiline and its (S) enantiomer increase survival and prevent stroke in salt-loaded stroke-prone spontaneously hypertensive rats", JOURNAL OF NEURAL TRANSMISSION, SPRINGER VERLAG, VIENNA, AT, vol. 108, no. 8-9, 1 January 2001 (2001-01-01), pages 909-923, XP002429255, ISSN: 0300-9564, DOI: 10.1007/S007020170012
- M. NAOI ET AL: "Anti-apoptotic function of propargylamine inhibitors of type-B monoamine oxidase", INFLAMMOPHARMACOLOGY, vol. 11, no. 2, 1 June 2003 (2003-06-01), pages 175-181, XP55002176, ISSN: 0925-4692, DOI: 10.1163/156856003765764344
- FUZHONG QIN ET AL: "Selegiline attenuates cardiac oxidative stress and apoptosis in heart failure: association with improvement of cardiac function.", EUROPEAN JOURNAL OF PHARMACOLOGY, vol. 461, no. 2-3, 1 February 2003 (2003-02-01), pages 149-58, XP55002162, ISSN: 0014-2999
- I. R. KOVELMAN, A. I. TOCHILKIN AND B. Z. GORKIN: "Structure and activity of irreversible inhibitors of monoamine oxidase", KHIMIKO-FARMATSEVTICHESKII ZHURNAL (PHARMACEUTICAL CHEMISTRY JOURNAL), vol. 24, no. 6, June 1990 (1990-06), pages 379-390, XP002512848,
- MOUSSA B H YOUDIM ET AL: "Rasagiline N-propargyl-1R(+)-aminoindan|, a selective and potent inhibitor of mitochondrial monoamine oxidase B", BRITISH JOURNAL OF PHARMACOLOGY, vol. 132, no. 2, 1 January 2001 (2001-01-01), pages 500-506, XP55002150, ISSN: 0007-1188, DOI: 10.1038/sj.bjp.0703826

## Description

### FIELD AND BACKGROUND OF THE INVENTION

The present invention relates to compositions for use in the treatment of cardiovascular disorders and diseases.

### Cardiovascular Disorders and Diseases

Cardiovascular disorders and diseases and their associated complications are a principal cause of disabilities and deaths of individuals in the United States and Western Europe. For example, in recent years more than 500,000 deaths have occurred annually in the United States alone as a result of coronary artery disease, and an additional 700,000 patients have been hospitalized for myocardial infarction.

Ischemic heart disease (IHD) is the most common, serious, chronic, life-threatening illness among the cardiovascular disorders and diseases. Ischemia, reduced myocardial perfusion, which causes lack of oxygen (hypoxia) as well as other metabolic changes, is the most common effect resulting from an inadequate blood flow through the coronary arteries, which are the blood suppliers of the heart. The most common cause of myocardial ischemia is the atherosclerotic disease of epicardial coronary arteries. The plaques consist of subintimal collections of fat, cells, and debris, which develop at irregular rates in different segments of the epicardial coronary tree, and lead eventually to segmental reductions in cross-sectional area (stenosis). When the coronary artery cross-section area is reduced by ∼75 %, a full range of increases in flow to meet increased myocardial demand is not possible. When the luminal area is reduced by more than 80%, blood flow at rest may be reduced, and further minor decreases in the stenotic orifice can reduce coronary flow dramatically and cause myocardial ischemia and infarction. This situation impairs myocardial contractility during exercise, creating the chest angina. Critical stenosis of the coronaries can cause chest angina even at rest, implying that the myocardium is suffering from lack of perfusion. The most serious complication of ischemic heart disease is acute myocardial infarction (AMI), which is one of the most common diagnoses in hospitalized patients. AMI generally occurs when coronary blood flow decreases abruptly after a thrombotic occlusion of a coronary artery, previously narrowed by atherosclerotic plaque. Although the mortality rate after admission for AMI has declined by about 30% over the last two decades, approximately 1 of every 25 patients who survives the initial hospitalization dies in the first year after AMI. The first step is the dissection of the atherosclerotic plaque, which causes the exposure of the thrombogenic plaque core to the blood. Because of its high thrombogenicity, a thrombus consists mainly of fibrin and activated thrombocyte is rapidly growing from the plaque core. Consequently, blood flow is seriously disturbed until there is no sufficient blood flow to the myocardium and an infarction begins due to lack of perfusion of oxygen.

There are various insults which cause the myocardial damage during myocardial ischemia and infarction. Lack of adequate perfusion to the heart tissue may result in: (i) lack of oxygen (hypoxia); (ii) growth factors and nutrients deprivation (e.g., IGF-1, insulin, glucose); (iii) acidosis (lactic acid production); (iv) hyperkalemia (due to environmental acidosis and cell damage); and/or (v) during ischemia, but mostly following reperfusion (resumption of the blood flow to the ischemic tissue), reactive oxygen species (ROS) such as H₂O₂, O_{2'}⁻ OH⁻ are created by the ischemic cells and by damage to neighboring cells. Each and every of these insults has been shown to exert damage to cardiomyocytes both *in vivo* and *in vitro.*

Two main cellular death types occur in nature: necrosis and apoptosis. While necrosis is a random process, often initiated by hostile environmental stimuli, apoptosis is a programmed cell death in which distinct intracellular signaling pathways are activated. Apoptosis is a fundamental physiological and pathologic mechanism that allows elimination of no-longer useful cells during embryogenesis, or of aged or damaged cells during life. Unlike necrosis, which involves large number of cells, apoptosis usually affects small number of cells without inflammation. In apoptosis, the nuclear DNA is "digested" into small fragments by special DNAses, while cytoskeletal and myofibrillar proteins are degraded by specialized proteases as well. At the final stages, the cell dissolves into a characteristic membrane bound vesicles (apoptotic bodies), which are quickly phagocyted by phagocytic neighboring cells.

Cells can undergo apoptosis caused by intrinsic stimuli, e.g. hypoxia, ROS, chemotherapies, or by extrinsic stimuli, mainly referred to activation of death receptors such as TNF-α and Fas.

Fas is a ubiquitous cell-surface receptor involved in apoptosis initiation. Fas belongs to the TNF/NGF superfamily, and is activated by Fas Ligand (FasL), which may cause apoptosis in Fas-bearing cells (Berke, 1997). It appears that while healthy cardiomyocytes are resistant to Fas-mediated apoptosis, during cardiac pathologies cardiomyocytes become sensitive to Fas-mediated apoptosis. Recent studies suggest that in several important heart diseases such as myocarditis, hypertrophy, ischemia, ischemia/reperfusion and heart failure, Fas activation results in apoptotic as well as in non-apoptotic effects, both contributing to cardiac dysfunction (Haunstetter and Izumo, 1998; Binah, 2000). Recent studies have shown that Fas activation is involved not only in myocardial pathologies inflicted by immune effectors (CTLs) such as transplant rejection, myocarditis and the resulting dilated cardiomyopathy (Binah, 2000; Hershkowitz et al., 1987), but also in lymphocyte-independent diseases such as ischemia/reperfusion injuries (Fliss and Gattinger, 1996; Yaoita et al., 1998; Jeremias et al., 2000). In this regard, it was recently proposed that FasL can be cleaved by a metalloprotease to form soluble FasL (sFasL), which can cause apoptosis in susceptible cells. Therefore, sFasL, which may be secreted from the failing heart and is elevated in patients with advanced congestive heart failure (Yamaguchi et al., 1999), is a potential contributor to apoptosis in this wide-spread heart pathology.

Programmed cell death (apoptosis) is recognized, increasingly, as a contributing cause of cardiac myocyte loss with ischemia/reperfusion injury, myocardial infarction, and long-standing heart failure.

### Propargylamine and propargylamine derivatives

Several propargylamine derivatives have been shown to selectively inhibit monoamine oxidase (MAO)-B and/or MAO-A activity and, thus to be suitable for treatment of neurodegenerative diseases such as Parkinson's and Alzheimer's disease. In addition, these compounds have been further shown to protect against neurodegeneration by preventing apoptosis.

Rasagiline, R(+)-N-propargyl-1-aminoindan, a highly potent selective irreversible monoamine oxidase (MAO)-B inhibitor, has been shown to exhibit neuroprotective activity and antiapoptotic effects against a variety of insults in cell cultures and in vivo and has finished recently the phase III clinical trials for Parkinson's disease.

R(+)-N-propargyl-1-aminoindan and pharmaceutically acceptable salts thereof were first disclosed in US Patents US 5,387,612, US 5, 453,446, US 5,457,133, US 5,576,353, US 5,668,181, US 5,786,390, US 5,891,923, and US 6,630,514 as useful for the treatment of Parkinson's disease, memory disorders, dementia of the Alzheimer type, depression, and the hyperactive syndrome. The 4-fluoro-, 5-fluoro- and 6-fluoro-N-propargyl-1-aminoindan derivatives were disclosed in US 5,486,541 for the same purposes.

US 5,519,061, US 5,532,415, US 5,599,991, US 5,744,500, US 6,277,886, US 6,316,504, US 133, US 5,576,353, US 5,668,181, US 5,786,390, US 5,891,923, and US 6,630,514 disclose R(+)-N-propargyl-1-aminoindan and pharmaceutically acceptable salts thereof as useful for treatment of additional indications, namely, an affective illness, a neurological hypoxia or anoxia, neurodegenerative diseases, a neurotoxic injury, stroke, brain ischemia, a head trauma injury, a spinal trauma injury, schizophrenia, an attention deficit disorder, multiple sclerosis, and withdrawal symptoms.

US 6,251,938 describes N-propargyl-phenylethylamine compounds, and US 6,303,650, US 6,462,222 and US 6,538,025 describe N-propargyl-1-aminoindan and N-propargyl-1-aminotetralin compounds, said to be useful for treatment of depression, attention deficit disorder, attention deficit and hyperactivity disorder, Tourette's syndrome, Alzheimer's disease and other dementia such as senile dementia, dementia of the Parkinson's type, vascular dementia and Lewy body dementia.

The first compound found to selectively inhibit MAO-B was R-(-)-N-methyl-N-(prop-2-ynyl)-2-aminophenylpropane, also known as L-(-)-deprenyl, R-(-)-deprenyl, or selegiline. In addition to Parkinson's disease, other diseases and conditions for which selegiline is disclosed as being useful include: drug withdrawal (WO 92/21333, including withdrawal from psychostimulants, opiates, narcotics, and barbiturates); depression (US 4,861,800); Alzheimer's disease and Parkinson's disease, particularly through the use of transdermal dosage forms, including ointments, creams and patches; macular degeneration (US 5,242,950); age-dependent degeneracies, including renal function and cognitive function as evidenced by spatial learning ability (US 5,151,449); pituitary-dependent Cushing's disease in humans and nonhumans (US 5,192,808); immune system dysfunction in both humans (US 5,387,615) and animals (US 5,276,057); age-dependent weight loss in mammals (US 5,225,446); schizophrenia (US 5,151,419); and various neoplastic conditions including cancers, such as mammary and pituitary cancers. WO 92/17169 discloses the use of selegiline in the treatment of neuromuscular and neurodegenerative disease and in the treatment of CNS injury due to hypoxia, hypoglycemia, ischemic stroke or trauma. In addition, the biochemical effects of selegiline on neuronal cells have been extensively studied (e.g., see Tatton, et al., 1991 and 1993). US 6,562,365 discloses the use of desmethylselegiline for selegiline-responsive diseases and conditions.

US 5,169,868, US 5,840,979 and US 6,251,950 disclose aliphatic propargylamines as selective MAO-B inhibitors, neuroprotective and cellular rescue agents. The lead compound, (R)-N-(2-heptyl)methyl-propargylamine (R-2HMP), has been shown to be a potent MAO-B inhibitor and antiapoptotic agent (Durden et al., 2000).

Propargylamine was reported many years ago to be a mechanism-based inhibitor of the copper-containing bovine plasma amine oxidase (BPAO), though the potency was modest. US 6,395,780 discloses propargylamine as a weak glycine-cleavage system inhibitor.

Eliash, Speiser, and Cohen found that rasagiline and its (S) enantiomer increase survival and prevent stroke in salt-loaded stroke-prone spontaneously hypertensive rats (J. Neural Transm. (2001), 108, pp 909-923).

Naoi, Maruyama, Youdim, Yu, and Boulton studied the anti-apoptotic function of propargylamine inhibitors of type-B monoamine oxidase (InflammoPharmacology (2003), 11, pp 175-181).

Kovel'man, Tochilkin, and Gorkin reviewed the structure and activity of irreversible inhibitors of monoamine oxidase (Khimiko-Farmatsevticheskii Zhurnal (1990), 24, pp 4-12).

Fuzhong et al. found that selegiline attenuates cardiac oxidative stress and apoptosis in heart failure which is associated with improvement of cardiac function (European Journal of Pharmacology (2003), 461, pp. 149-158).

Youdim, Gross, and Finberg studied rasagiline (N-propargyl-1R(+)-aminoindan) and found that it is a selective and potent inhibitor of mitochondrial monoamine oxidase B (British Journal of Pharmacology (2001), 132, pp. 500-506).

### SUMMARY OF THE INVENTION

The present invention is as described in the claims.

The compositions of the invention are suitable for preventing and/or treating myocardial infarction, myocardial ischemia, or myocardial ischemia and reperfusion.

### BRIEF DESCRIPTION OF THE FIGURES

**Fig. 1** depicts apoptosis induced in H9c2 heart cell line by means of recombinant Fas ligand (rFasL). The apoptotic cells detected by DAPI staining are marked by the arrows (right side).
**Fig. 2** shows that rasagiline blocks Fas-mediated apoptosis in H9c2 cells.
**Fig. 3** shows that rasagiline protects against serum starvation-induced apoptosis in H9c2 cells.
**Fig. 4** shows that rasagiline protects against serum starvation-mediated but not H₂O₂- induced apoptosis in H9c2 cells. * compared to control. ** compared to serum starvation (p<0.05).
**Fig. 5** shows that propargylamine blocks Fas-mediated hypertrophy in cultured neonatal rat ventricular myocytes. The top panel depicts representative atrial natriuretic peptide (ANP) mRNA blots in Control, rFasL, and in rFasL + propargylamine. The lower panel depicts the summary of three experiments. Hypertrophy was expressed as the ratio between ANP and actin. * P < 0.05 vs. control.

### DETAILED DESCRIPTION OF THE INVENTION

The present invention relates to the use of S-(-)-N-propargyl-1-aminoindan or a pharmaceutically acceptable salt thereof for the preparation of a pharmaceutical composition for prevention and/or treatment of a cardiovascular disease or disorder selected from myocardial infarction, myocardial ischemia, or myocardial ischemia and reperfusion.

The present invention further relates to pharmaceutical composition comprising S-(-)-N-propargyl-1-aminoindan or a pharmaceutically acceptable salt thereof for use in the prevention and/or treatment of a cardiovascular disease or disorder selected from myocardial infarction, myocardial ischemia, or myocardial ischemia and reperfusion.

Disclosed herein is that the active agent used can be propargylamine or a pharmaceutically acceptable salt thereof. The use of any physiologically acceptable salt of propargylamine is disclosed such as the hydrochloride, hydrobromide, sulfate, mesylate, esylate, tosylate, sulfonate, phosphate, or carboxylate salt. It is preferred that propargylamine hydrochloride and propargylamine mesylate are used.

Also disclosed herein is that the active agent used can be N-propargyl-1-aminoindan, either in its racemic form (described, for example, in US 6,630,514) or as the R-enantiomer R(+)-N-propargyl-1-aminoindan (described, for example, in US 5,387,612) or as the S-enantiomer S-(-)-N-propargyl-1-aminoindan (described, for example, in US 6,277,886). It is preferred that the active agent is rasagiline, the R(+)-N-propargyl-1-aminoindan.

Disclosed herein is that the active agent can be a pharmaceutically acceptable salt ofN-propargyl-1-aminoindan or of an enantiomer thereof including, but not limited to, the mesylate, maleate, fumarate, tartrate, hydrochloride, hydrobromide, esylate, p-toluenesulfonate, benzoate, acetate, phosphate and sulfate salts. It is preferred that the salt is a pharmaceutically acceptable salt of R(+)-N-propargyl-1-aminoindan such as, but not limited to, the mesylate salt (described, for example, in US 5,532,415), the esylate and the sulfate salts (both described, for example, in US 5,599,991), and the hydrochloride salt (described, for example, in US 6,630,514) of R(+)-N-propargyl-1-aminoindan.

It is also disclosed that the active agent can be an analog of N-propargyl-1-aminoindan, an enantiomer or a pharmaceutically acceptable salt thereof. The analogs can be the compounds described in US 5,486,541 such as, but not limited to, the compounds 4-fluoro-N-propargyl-1-aminoindan, 5-fluoro-N-propargyl-1-aminoindan, 6-fluoro-N-propargyl-1-aminoindan, an enantiomer thereof and pharmaceutically acceptable addition salts thereof. The analogs can also be the compounds described in US 6,251,938 such as, but not limited to, the compounds (rac)-3-(N-methyl,N-propyl-carbamyloxy)-α-methyl-N'-propargyl phenethylamine HCl; (rac)-3-(N,N-dimethyl-carbamyloxy)- α-methyl-N'-methyl, N'-propargyl phenethylamine HCl; (rac)-3-(N-methyl,N-hexyl-carbamyloxy)-α-methyl-N'-methyl, N'-propargyl phenethylamine mesylate; (rac)-3-(N-methyl,N-cyclohexyl-carbamyloxy)-α-methyl-N'-methyl,N'-propargylphenethyl HCl; and (S)-3-(N-methyl, N-hexyl-carbamyloxy)-α-methyl-N'-methyl,N'-propargyl phenethylamine ethane-sulfonate. Alternatively, the analogs can be the compounds described in US 6,303,650 such as, but not limited to, the compounds (rac) 6-(N-methyl, N-ethyl-carbamyloxy)-N'-propargyl-1-aminoindan HCl; (rac) 6-(N,N-dimethyl, carbamyloxy)-N'-methyl-N'-propargyl-1-aminoindan HCl; (rac) 6-(N-methyl, N-ethyl-carbamyloxy-N'-propargyl-1-aminotetralin HCl; (rac) 6-(N,N-dimethyl-thiocarbamyloxy)-1-aminoindan HCl; (rac) 6-(N-propyl-carbamyloxy-N'-propargyl-1-aminoindan HCl; (rac) 5-chloro-6-(N-methyl, N-propyl-carbamyloxy)-N'-propargyl-1-aminoindan HCl; (S)-6-(N-methyl), N-propyl-carbamyloxy)-N'-propargyl-1-aminoindan HCl; and (R)-6-(N-methyl, N-ethyl-carbamyloxy)-N'-propargyl-1-aminoindan hemi-(L)-tartrate, and 6-(N-methyl, N-ethyl-carbamyloxy)-N'-methyl,N'-propargyl-1-aminoindan described in US 6,462,222.

Also disclosed is that the active agent can be an aliphatic propargylamine described in US 5,169,868, US 5,840,979 and US 6,251,950 such as, but not limited to, the compounds N-(1-heptyl)propargylamine; N-(1-octyl)propargylamine; N-(1-nonyl) propargylamine; N-(1-decyl)propargylamine; N-(1-undecyl)propargylamine: N-(1-dodecyl) propargylamine; R-N-(2-butyl) propargylamine; R-N-(2-pentyl) propargylamine; R-N-(2-hexyl)propargylamine; R-N-(2-heptyl)propargylamine; R-N-(2-octyl) propargylamine; R-N-(2-nonyl) propargylamine; R-N-(2-decyl) propargylamine, R-N-(2-undecyl)propargylamine; R-N-(2-dodecyl)propargylamine: N-(1-butyl)-N-methylpropargylamine; N-(2-butyl)-N-methylpropargylamine ; N-(2-pentyl)-N-methylpropargylamine; N-(1-pentyl)-N-methylpropargylamine; N-(2-hexyl)-N-methylpropargylamine; N-(2-heptyl)-N-methylpropargylamine; N-(2-decyl)-N-methylpropargylamine; N-(2-dodecyl)-N-methylpropargylamine; R(-)-N-(2-butyl)-N-methylpropargylamine; or a pharmaceutically acceptable salt thereof.

Disclosed herein is that the active agent can be selegiline, desmethylselegiline or norprenyl, pargyline or chlorgyline, or the compound (N-methyl-N-propargyl-10-aminomethyl-dibenzo[b,f]oxepin (known as CGP 3466, described in Zimmermann et al., 1999).

All the US patents and other publications mentioned hereinabove are hereby incorporated by reference in their entirety as if fully disclosed herein.

In another aspect, the present invention provides a pharmaceutical composition for prevention and/or treatment of a cardiovascular disorder or disease selected from myocardial infarction, myocardial ischemia, or myocardial ischemia and reperfusion, comprising a pharmaceutically acceptable carrier and S-(-)-N-propargyl-1-ammoindan, or a pharmaceutically acceptable salt thereof as described above.

The pharmaceutical composition provided by the present invention may be in solid, semisolid or liquid form and may further include pharmaceutically acceptable fillers, carriers or diluents, and other inert ingredients and excipients. The composition can be administered by any suitable route, e.g. intravenously, orally, parenterally, rectally, or transdermally. The dosage will depend on the state of the patient and severity of the disease and will be determined as deemed appropriate by the practitioner.

In one embodiment, the pharmaceutically acceptable carrier is a solid and the pharmaceutical composition is in a suitable form for oral administration including tablets, compressed or coated pills, dragees, sachets, hard or soft gelatin capsules, and sublingual tablets. In a more preferred embodiment, the pharmaceutical composition is a tablet containing an amount of the active agent in the range of about 0.1 - 100 mg, preferably from about 1 mg to about 10 mg.

In another embodiment, the pharmaceutically acceptable carrier is a liquid and the pharmaceutical composition is an injectable solution. The amount of the active agent in the injectable solution is in the range of from about 0.1 mg/ml to about 100 mg/ml, more preferably 1 mg/ml to about 10 mg/ml.

For parenteral administration the invention provides ampoules or vials that include an aqueous or non-aqueous solution or emulsion. For rectal administration there are provided suppositories with hydrophilic or hydrophobic (gel) vehicles.

The compositions of the invention are for preventing and/or treating myocardial infarction, myocardial ischemia, myocardial, or ischemia and reperfusion. In preferred embodiments, the cardiovascular disorder is ischemia.

The dosage and frequency of administration of the drug will depend from the age and condition of the patient, type of disorder and its severity, and will be determined according to the physician's judgment. It can be presumed that for preventive treatment of subjects susceptible to a cardiovascular disorder or disease lower doses will be needed while higher doses will be administered in acute cases.

In one embodiment of the invention, the active agent is administered alone. In other embodiments of the invention, the active agent is administered in combination with another known cardiovascular drug, either before, simultaneously or after said other cardiovascular drug.

The disclosure content of this document is summarized by the following items:
1. A method for treatment of a subject susceptible to or suffering from a cardiovascular disorder or disease which comprises administering to the subject an amount of an active agent selected from the group consisting of propargylamine, a propargylamine derivative, and a pharmaceutically acceptable salt thereof, effective to treat the subject.
2. A method according to item 1 for preventing and/or treating congestive heart failure, cardiac hypertrophy including atrial and ventricular hypertrophy, myocardial infarction, myocardial ischemia, myocardial ischemia and reperfusion, or arrhythmias.
3. A method according to item 1 or 2 wherein said active agent is selected from the group consisting of N-propargyl-1-aminoindan, an enantiomer thereof, an analog thereof, and a pharmaceutically acceptable salt of the aforesaid.
4. A method according to item 3 wherein said active agent is racemic N-propargyl-1-aminoindan.
5. A method according to item 3 wherein said active agent is the enantiomer R(+)-N-propargyl-1-aminoindan.
6. A method according to item 3 wherein said active agent is the enantiomer is S-(-)-N-propargyl- I -aminoindan.
7. A method according to item 3 wherein said active agent is a pharmaceutically acceptable salt of R(+)-N-propargyl-1-aminoindan.
8. A method according to item 7 wherein said pharmaceutically acceptable salt is selected from the group consisting of the mesylate salt; the esylate salt; the sulfate salt; and the hydrochloride salt of R(+)-N-propargyl-1-aminoindan.
9. A method according to item 3 wherein said analog of N-propargyl-1-aminoindan is selected from the group consisting of 4-fluoro-N-propargyl-1-aminoindan, 5-fluoro-N-propargyl-1-aminoindan, 6-fluoro-N-propargyl-1-aminoindan, an enantiomer thereof and pharmaceutically acceptable addition salts thereof.
10. A method according to item 3 wherein said analog of N-propargyl-1-aminoindan is selected from the group consisting of: (rac)-3-(N-methyl,N-propyl-carbamyloxy)-α-methyl-N'-propargyl phenethylamine HCl; (rac)-3-(N,N-dimethyl-carbamyloxy)- α-methyl-N'-methyl, N'-propargyl phenethylamine HCl; (rac)-3-(N-methyl,N-hexyl-carbamyloxy)-α-methyl-N'-methyl, N'-propargyl phenethylamine mesylate; (rac)-3-(N-methyl,N-cyclohexyl-carbamyloxy)-α-methyl-N'-methyl,N'-propargyl phenethylamine HCl; and (S)-3-(N-methyl, N-hexyl-carbamyloxy)-α-methyl-N'-methyl,N'-propargyl phenethylamine ethanesulfonate.
11. A method according to item 3 wherein said analog of N-propargyl-1-aminoindan is selected from the group consisting of: (rac) 6-(N-methyl, N-ethyl-carbamyloxy)-N'-propargyl-1-aminoindan HCl; (rac) 6-(N,N-dimethyl, carbamyloxy)-N'-methyl-N'-propargyl-1-aminoindan HCl; (rac) 6-(N-methyl, N-ethyl-carbamyloxy-N'-propargyl-1-aminotetralin HCl; (rac) 6-(N,N-dimethyl-thiocarbamyloxy)-1-aminoindan HCl; (rac) 6-(N'-propyl-carbamyloxy-N'-propargyl-1-aminoindan, HCl; (rac) 5-chloro-6-(N-methyl, N-propyl-carbamyloxy)-N'-propargyl-1-aminoindan HCl; (S)-6-(N-methyl), N-propyl-carbamyloxy)-N'-propargyl-1-aminoindan HCl; and (R)-6-(N-methyl, N-ethyl-carbamyloxy)-N'-propargyl-1-aminoindan hemi-(L)-tartrate. 6 and 6-(N-methyl, N-ethyl-carbamyloxy)-N'-methyl,N'-propargyl-1-aminoindan.
12. A method according to item 1 or 2 wherein said active agent is an aliphatic propargylamine.
13. A method according to item 12 wherein said aliphatic propargylamine is selected from the group consisting of the compounds N-(1-heptyl)propargylamine or a propargylamine; N-(1-octyl)propargylamine; N-(1-nonyl) propargylamine; N-(1-decyl)propargyl-amine; N-(1-undecyl)propargylamine: N-(1-dodecyl) propargylamine; R-N-(2-butyl)propargylamine; R-N-(2-pentyl) propargylamine; R-N-(2-hexyl) propargylamine; R-N-(2-heptyl)propargylamine; R-N-(2-octyl) propargylamine; R-N-(2-nonyl)propargylamine; R-N-(2-decyl) propargylamine, R-N-(2-undecyl) propargylamine; R-N-(2-dodecyl)propargylamine: N-(1-butyl)-N-methylpropargyl-amine; N-(2-butyl)-N-methylpropargylamine; N-(2-pentyl)-N-methylpropargyl-amine; N-(1-pentyl)-N-methylpropargylamine; N-(2-hexyl)-N-methylpropargyl-amine; N-(2-heptyl)-N-methylpropargylamine; N-(2-decyl)-N-methylpropargyl-amine; N-(2-dodecyl)-N-methylpropargylamine; R(-)-N-(2-butyl)-N-methyl-propargylamine; or a pharmaceutically acceptable salt thereof.
14. A method according to item 1 or 2 wherein said active agent is selected from the group consisting of selegiline, desmethylselegiline, pargyline and chlorgyline.
15. A method according to item 1 or 2 wherein said active agent is the compound (N-methyl-N-propargyl-10-aminomethyl-dibenzo[b,f]oxepin.
16. A method according to item 1 or 2 wherein said active agent is propargylamine or a pharmaceutically acceptable salt thereof.
17. A method according to item 1 or 2 for protecting ventricular muscle from apoptosis, wherein said cardiovascular disorder or disease is ischemia/reperfusion injury, myocardial infarction, and long-standing heart failure.
18. A method according to item 17, wherein said apoptosis is Fas-mediated apoptosis.
19. A pharmaceutical composition for prevention and/or treatment of a cardiovascular disorder or disease comprising a pharmaceutically acceptable carrier and an active agent selected from the group consisting of propargylamine, a propargylamine derivative and a pharmaceutically acceptable salt thereof.
20. A pharmaceutical composition according to item 19 for preventing and/or treating congestive heart failure, cardiac hypertrophy including atrial and ventricular hypertrophy, myocardial infarction, myocardial ischemia, myocardial ischemia and reperfusion, or arrhythmias.
21. A pharmaceutical composition according to item 19 or 20 wherein said active agent is selected from the group consisting of N-propargyl-1-aminoindan, an enantiomer thereof, an analog thereof, and a pharmaceutically acceptable salt of the aforesaid.
22. A pharmaceutical composition according to item 21 wherein said active agent is racemic N-propargyl-1-aminoindan.
23. A pharmaceutical composition according to item 21 wherein said active agent is the enantiomer R(+)-N-propargyl-1-aminoindan.
24. A pharmaceutical composition according to item 21 wherein said active agent is the enantiomer is S-(-)-N-propargyl-1-aminoindan.
25. A pharmaceutical composition according to item 21 wherein said active agent is a pharmaceutically acceptable salt of R(+)-N-propargyl-1-aminoindan.
26. A pharmaceutical composition according to item 25 wherein said pharmaceutically acceptable salt is selected from the group consisting of the mesylate salt; the esylate salt; the sulfate salt; and the hydrochloride salt of R(+)-N-propargyl-1 -aminoindan.
27. A pharmaceutical composition according to item 21 wherein said analog of N-propargyl-1-aminoindan is selected from the group consisting of 4-fluoro-N-propargyl-1-aminoindan, 5-fluoro-N-propargyl-1-aminoindan, 6-fluoro-N-propargyl-1-amino- indan, an enantiomer thereof and pharmaceutically acceptable addition salts thereof.
28. A pharmaceutical composition according to item 21 wherein said analog of N-propargyl-1-aminoindan is selected from the group consisting of: (rac)-3-(N-methyl,N-propyl-carbamyloxy)-a-methyl-N'-propargyl phenethylamine HCl; (rac)-3-(N,N-dimethyl-carbamyloxy)- α-methyl-N'-methyl, N'-propargyl phenethylamine HCl; (rac)-3-(N-methyl,N-hexyl-carbamyloxy)-a-methyl-N'-methyl, N'-propargyl phenethylamine mesylate; (rac)-3-(N-methyl,N-cyclohexyl-carbamyloxy)-α-methyl-N'-methyl,N'-propargyl phenethylamine HCl; and (S)-3-(N-methyl, N-hexyl-carbamyloxy)-α-methyl-N'-methyl,N'-propargyl phenethylamine ethane sulfonate.
29. A pharmaceutical composition according to item 21 wherein said analog of N-propargyl-1-aminoindan is selected from the group consisting of: (rac) 6-(N-methyl, N-ethyl-carbamyloxy)-N'-propargyl-1-aminoindan HCl; (rac) 6-(N,N-dimethyl, carbamyloxy)-N'-methyl-N'-propargyl-1-aminoindan HCl; (rac) 6-(N-methyl, N-ethyl-carbamyloxy-N'-propargyl-1-aminotetralin HCl; (rac) 6-(N,N-dimethyl-thiocarbamyloxy)-1-aminoindan HCl; (rac) 6-(N-propyl-carbamyloxy-N'-propargyl-1-aminoindan HCl; (rac) 5-chloro-6-(N-methyl, N-propyl-carbamyloxy)-N'-propargyl-1-aminoindan HCl; (S)-6-(N-methyl), N-propyl-carbamyloxy)-N'-propargyl-1-aminoindan HCl; and (R)-6-(N-methyl, N-ethyl-carbamyloxy)-N'-propargyl-1-aminoindan hemi-(L)-tartrate. 6 and 6-(N-methyl, N-ethyl-carbamyloxy)-N'-methyl,N'-propargyl-1-aminoindan.
30. A pharmaceutical composition according to item 19 or 20 wherein said active agent is an aliphatic propargylamine.
31. A pharmaceutical composition according to item 30 wherein said aliphatic propargylamine is selected from the group consisting of the compounds N-(1-heptyl)propargylamine; N-(1-octyl)propargylamine; N-(1-nonyl) propargylamine; N-(1-decyl)propargyl-amine; N-(1-undecyl)propargylamine: N-(1-dodecyl) propargylamine; R-N-(2-butyl)propargylamine; R-N-(2-pentyl) propargylamine; R-N-(2-hexyl) propargylamine; R-N-(2-heptyl)propargylamine; R-N-(2-octyl) propargylamine; R-N-(2-nonyl)propargylamine; R-N-(2-decyl) propargylamine, R-N-(2-undecyl) propargylamine; R-N-(2-dodecyl)propargylamine: N-(1-butyl)-N-methylpropargyl-amine; N-(2-butyl)-N-methylpropargylamine ; N-(2-pentyl)-N-methylpropargyl-amine; N-(1-pentyl)-N-methylpropargylamine; N-(2-hexyl)-N-methylpropargyl-amine; N-(2-heptyl)-N-methylpropargylamine; N-(2-decyl)-N-methylpropargyl-amine; N-(2-dodecyl)-N-methylpropargylamine; R(-)-N-(2-butyl)-N-methyl-propargylamine; or a pharmaceutically acceptable salt thereof.
32. A pharmaceutical composition according to item 19 or 20 wherein said active agent is selected from the group consisting of selegiline, desmethylselegiline, pargyline and chlorgyline.
33. A pharmaceutical composition according to item 19 or 20 wherein said active agent is the compound (N-methyl-N-propargyl-10-aminomethyl-dibenzo[b,f]oxepin.
34. A pharmaceutical composition according to item 19 or 20 wherein said active agent is propargylamine or a pharmaceutically acceptable salt thereof.
35. A pharmaceutical composition according to any one of items 19 to 34 for intravenous, oral, rectal, transdermal or parenteral administration.
36. Use of an active agent selected from propargylamine, a propargylamine derivative or a pharmaceutically acceptable salt thereof for the preparation of a pharmaceutical composition for prevention and/or treatment of a cardiovascular disease or disorder.
37. The use according to item 36 for preventing and/or treating congestive heart failure, cardiac hypertrophy including atrial and ventricular hypertrophy, myocardial infarction, myocardial ischemia, myocardial ischemia and reperfusion, or arrhythmias.
38. The use according to item 36 or 37 wherein said active agent is selected from the group consisting of N-propargyl-1-aminoindan, an enantiomer thereof, an analog thereof, and a pharmaceutically acceptable salt of the aforesaid.
39. The use according to item 38 wherein said active agent is racemic N-propargyl-1-aminoindan.
40. The use according to item 38 wherein said active agent is the enantiomer R(+)-N-propargyl-1-aminoindan.
41. The use according to item 38 wherein said active agent is the enantiomer is S-(-)-N-propargyl-1-aminoindan.
42. The use according to item 38 wherein said active agent is a pharmaceutically acceptable salt of R(+)-N-propargyl-1-aminoindan.
43. The use according to item 42 wherein said pharmaceutically acceptable salt is selected from the group consisting of the mesylate salt; the esylate salt; the sulfate salt; and the hydrochloride salt of R(+)-N-propargyl-1-aminoindan.
44. The use according to item 38 wherein said analog of N-propargyl-1-aminoindan is selected from the group consisting of 4-fluoro-N-propargyl-1-aminoindan, 5-fluoro-N-propargyl-1-aminoindan, 6-fluoro-N-propargyl-1-aminoindan, an enantiomer thereof and pharmaceutically acceptable addition salts thereof.
45. The use according to item 38 wherein said analog of N-propargyl-1-aminoindan is selected from the group consisting of: (rac)-3-(N-methyl,N-propyl-carbamyloxy)-a-methyl-N'-propargyl phenethylamine HCl; (rac)-3-(N,N-dimethyl-carbamyloxy)- α-methyl-N'-methyl, N'-propargyl phenethylamine HCl; (rac)-3-(N-methyl,N-hexyl-carbamyloxy)-α-methyl-N'-methyl, N'-propargyl phenethylamine mesylate; (rac)-3 -(N-methyl,N-cylohexyl-carbamyloxy)-α-methyl-N'-methyl,N'-propargyl phenethylamine HCl; and (S)-3-(N-methyl, N-hexyl-carbamyloxy)-α-methyl-N'-methyl,N'-propargyl phenethylamine ethanesulfonate.
46. The use according to item 38 wherein said analog of N-propargyl-1-aminoindan is selected from the group consisting of: (rac) 6-(N-methyl, N-ethyl-carbamyloxy)-N'-propargyl-1-aminoindan HCl; (rac) 6-(N,N-dimethyl, carbamyloxy)-N'-methyl-N'-propargyl-1-aminoindan HCl; (rac) 6-(N-methyl, N-ethyl-carbamyloxy-N'-propargyl-1-aminotetralin HCl; (rac) 6-(N,N-dimethyl-thiocarbamyloxy)-1-aminoindan HCl; (rac) 6-(N-propyl-carbamyloxy-N'-propargyl-1-aminoindan HCl; (rac) 5-chloro-6-(N-methyl, N-propyl-carbamyloxy)-N'-propargyl-1-aminoindan HCl; (S)-6-(N-methyl), N-propyl-carbamyloxy)-N'-propargyl-1-aminoindan HCl; and (R)-6-(N-methyl, N-ethyl-carbamyloxy)-N'-propargyl-1-aminoindan hemi-(L)-tartrate. 6 and 6-(N-methyl, N-ethyl-carbamyloxy)-N'-methyl,N'-propargyl-1-aminoindan.
47. The use according to item 36 or 37 wherein said active agent is an aliphatic propargylamine.
48. The use according to item 47 wherein said aliphatic propargylamine is selected from the group consisting of the compounds N-(1-heptyl)propargylamine or a propargylamine; N-(1-octyl)propargylamine; N-(1-nonyl) propargylamine; N-(1-decyl)propargyl-amine; N-(1-undecyl)propargylamine: N-(1-dodecyl) propargylamine; R-N-(2-butyl)propargylamine; R-N-(2-pentyl) propargylamine; R-N-(2-hexyl) propargylamine; R-N-(2-heptyl)propargylamine; R-N-(2-octyl) propargylamine; R-N-(2-nonyl)propargylamine; R-N-(2-decyl) propargylamine, R-N-(2-undecyl) propargylamine; R-N-(2-dodecyl)propargylamine: N-(1-butyl)-N-methylpropargyl-amine; N-(2-butyl)-N-methylpropargylamine; N-(2-pentyl)-N-methylpropargyl-amine; N-(1-pentyl)-N-methylpropargylamine; N-(2-hexyl)-N-methylpropargyl-amine; N-(2-heptyl)-N-methylpropargylamine; N-(2-decyl)-N-methylpropargyl-amine; N-(2-dodecyl)-N-methylpropargylamine; R(-)-N-(2-butyl)-N-methyl-propargylamine; or a pharmaceutically acceptable salt thereof.
49. The use according to item 36 or 37 wherein said active agent is selected from the group consisting of selegiline, desmethylselegiline, pargyline and chlorgyline.
50. The use according to item 36 or 37 wherein said active agent is the compound (N-methyl-N-propargyl-10-aminomethyl-dibenzo[b,f]oxepin.
51. The use according to item 36 or 37 wherein said active agent is propargylamine or a pharmaceutically acceptable salt thereof.
52. An article of manufacture comprising packaging material and a pharmaceutical composition contained within the packaging material, said pharmaceutical composition comprising an active agent selected from propargylamine, a propargylamine derivative or a pharmaceutically acceptable salt thereof, and said packaging material includes a label that indicates that said agent is therapeutically effective for treating a cardiovascular disease or disorder.

The following examples illustrate certain features of the present invention but are not intended to limit the scope of the present invention.

### EXAMPLES

### Materials and Methods

***(i) Materials.*** Rasagiline and propargylamine were kindly donated by Teva Pharmaceutical Industries Ltd. (Petach Tikva, Israel).
***(ii)*** ***Cell line H9c2.*** Experiments were performed on the embryonic rat heart cell line H9c2. H9c2 cells were cultured in DMEM (Biological Industries, Beit-Haemek, Israel) supplemented with 10% FCS, 50 units/ml penicillin G, 50 µg/ml streptomycin sulfate, 2 mg/ml L-glutamine and sodium pyruvate. H9c2 cells were harvested by trypsinization, washed with PBS, diluted to a concentration of 5x10⁴ cells/ml with DMEM (high glucose) and cultured at 0.5 ml/well on sterile glass cover slips in 24-well plates.

### (iii) Protocols inducing apoptosis

(a) H₂O₂ Incubation protocol - To induce apoptosis, H9c2 cultures were exposed to H₂O₂ (0.5 µM) for 7 hours.
(b) Serum starvation - To induce apoptosis, H9c2 cultures were incubated in the culture medium containing 0% FCS for the indicated times.
(c) Activation of the Fas receptor - Fas activation was induced by incubating the cultures with recombinant human Fas Ligand (rFasL; 10 ng/ml) plus the enhancing antibody (1 µg/ml) for the indicated times, according to the manufacturer's recommendations (Alexis Biochemicals, San Diego, CA).

### (iv) Determination of Apoptosis by DAPI. Cultures were counterstained with DAPI to visualize the nuclear morphology. Cells were scored as apoptotic, only if they exhibited unequivocal nuclear chromatin condensation and fragmentation.

In the following experiments, our major aim was to determine whether rasagiline and propargylamine can provide protection against apoptosis induced by several means in the embryonic cardiac cell line H9c2 or in neonatal rat ventricular myocytes.

### Example 1. Rasagiline protects H9c2 heart cells against apoptosis induced by Fas activation (Reference)

The first apoptosis-inducing protocol tested was activation of the Fas receptor with recombinant Fas Ligand (rFasL) plus the enhancing antibody (Yaniv et al., 2002).

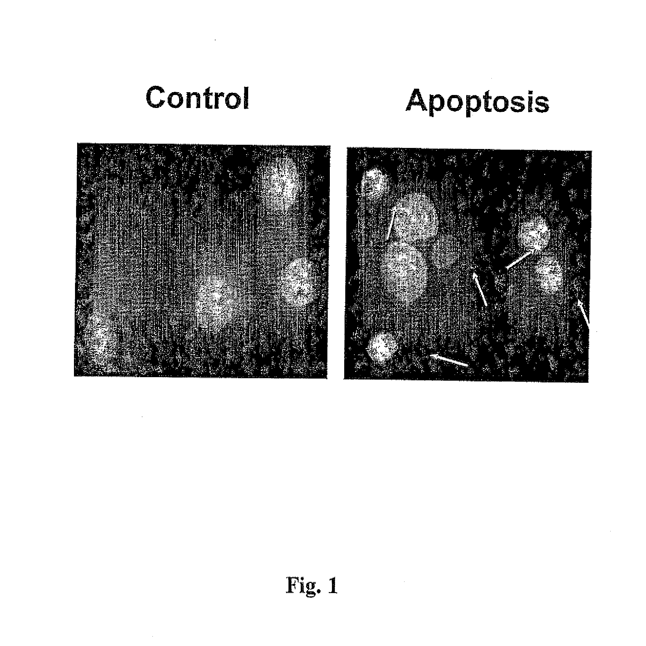

Cultures of embryonic rat heart cell line H9c2 were incubated with rFasL (10 ng/ml) and an enhancing antibody for periods of time of 9, 10 and 24 hours, and apoptosis measured thereafter. As shown in Fig. 1, Fas activation caused prominent apoptosis in H9c2 cells, as detected by the DAPI assay.

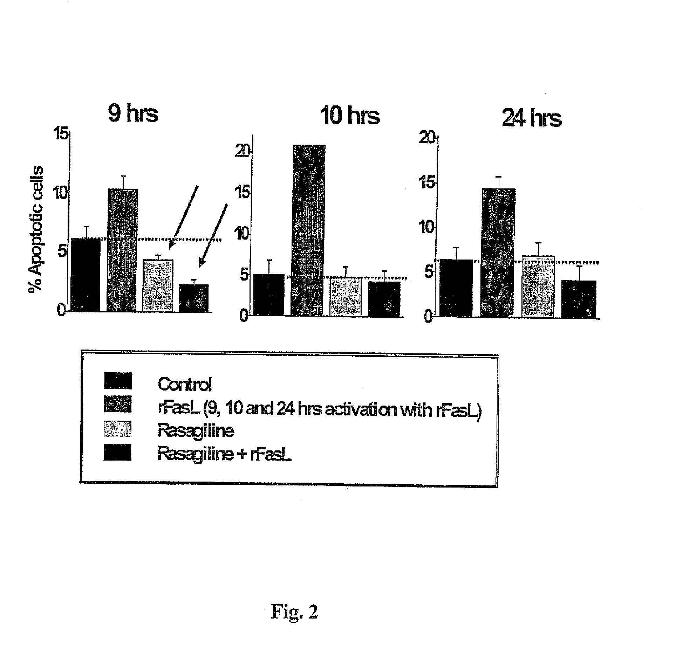

In order to determine whether rasagiline can prevent Fas-mediated apoptosis, the Fas receptor was activated for 9, 10 and 24 hours as described above. Rasagiline (10 µM) was introduced to the culture medium 16 hours before, and was present throughout the apoptosis-inducing protocol (n= 3 wells). As seen in Fig. 2, the maximal apoptotic effect (∼20% apoptosis) of Fas activation was attained at 10 hours incubation with rFasL. This apoptotic effect was completely prevented by rasagiline, demonstrating that rasagiline blocks Fas-mediated apoptosis.

### Example 2. Rasagiline protects cultured neonatal rat ventricular myocytes against apoptosis induced by Fas activation (Reference)

To test whether rasagiline can prevent the marked hypertrophy induced in cultured neonatal rat ventricular myocytes (for methods see: Yaniv et al, 2002), Fas was activated by 24 hours incubation with rFasL (10 ng/ml rFasL plus 1 µg/ml of the enhancer antibody). Hypertrophy was assessed by determining the mRNA levels (by means of RT-PCR) of the atrial natriuretic peptide (ANP), which is a most common molecular marker of hypertrophy. Rasagiline (10 µM/ml )was added to the culture 1 hour before Fas activation and remained in the medium throughout the 24 hours exposure to rFasL. In these preliminary experiments we have found that rasagiline prevented Fas-mediated hypertrophy, a finding which may have an important clinical significance.

### Example 3. Rasagiline protects H9c2 heart cells against apoptosis induced by serum starvation (Reference)

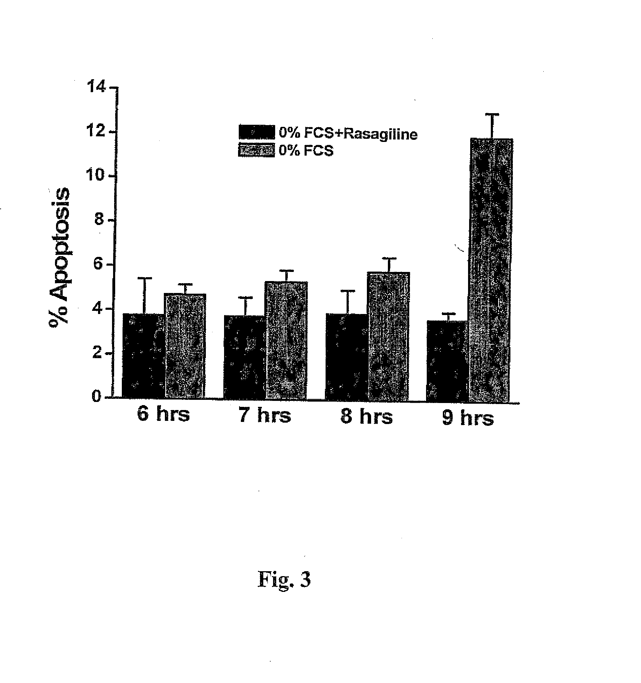

The next apoptosis-inducing stimulus tested was serum starvation (24 hrs, 0 % serum in the culture medium). To induce apoptosis, H9c2 cells were incubated in the culture medium containing 0 % FCS for 6, 7, 8 or 9 hours. Rasagiline was introduced to the culture medium 2 hours before inducing serum starvation and was present throughout the apoptosis-inducing protocol (n= 3 wells). As seen in Fig. 3, the most effective protocol was 9 hrs serum starvation, which caused 12% apoptosis. This effect was completely prevented by rasagiline (10 µM).

### Example 4. Rasagiline protects H9c2 heart cells against apoptosis induced by serum starvation but not H₂O₂-induced apoptosis (Reference)

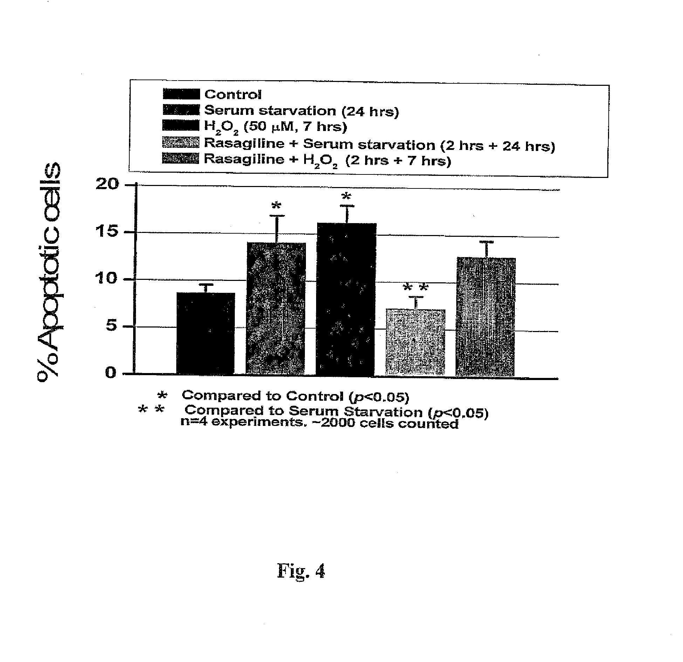

In another experiment, we repeated the serum starvation protocol, and also tested in the same cultures whether rasagiline can protect against H₂O₂-induced apoptosis. Rasagiline was introduced to the culture medium 2 hours before inducing serum starvation or adding H₂O₂, and was present throughout the apoptosis-inducing protocol (n= 4 experiments; about 2000 cells counted). As clearly shown in Fig. 4, rasagiline prevented the apoptosis induced by serum starvation (green bar), but not by H₂O₂ (gray bar).

In summary, rasagiline prevented apoptosis induced by Fas activation (as well as the hypertrophy) and by serum starvation, which may have an immense clinical importance for the prevention and treatment of major cardiac pathologies.

### Example 5. Propargylamine blocks hypertrophy induced by activation of the Fas receptor in cultures of neonatal rat ventricular myocytes (Reference)

To test whether propargylamine can prevent the marked hypertrophy induced in cultures of neonatal rat ventricular myocytes (for methods, see Yaniv et al., 2002), Fas was activated for 24 hours with 10 ng/ml rFasL plus 1 µg/ml enhancer antibody (Yaniv et al., 2002). Hypertrophy was assessed by determining the mRNA levels (by means of RT-PCR) of ANP, which is a most common molecular marker of hypertrophy. Ten µM of propargylamine were added to the appropriate culture 60 minutes before Fas activation.

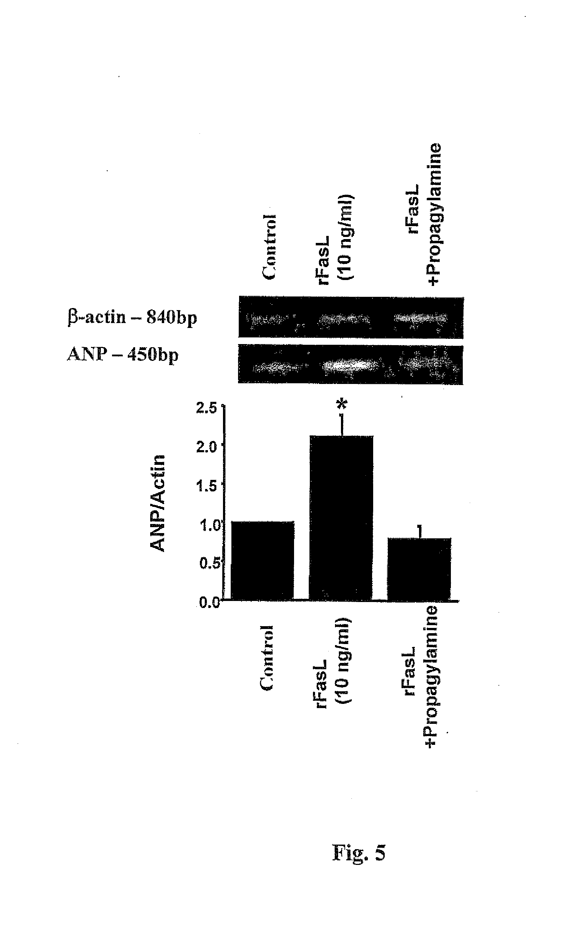

As shown in Fig. 5, ANP mRNA elevation induced by incubation with rFasL for 24 h was blocked by 10 µM of propargylamine. n = 3 experiments, **P* < 0.05 versus control. We conclude that propargylamine protects ventricular myocytes against hypertrophy caused by activation of the Fas receptor and this finding can have an important clinical significance.

### REFERENCES

Berke G. 1997. The Fas-based mechanism of lymphotoxicity. Human Immunol 54:1-7.
Binah O. 2000. Immune effector mechanisms in myocardial pathologies. Int J Mol Med 6:3-16.
Durden DA, Dyck LE, Davis BA, Liu YD, Boulton AA. 2000. Metabolism and pharmacokinetics, in the rat, of (R)-N-(2-Heptyl)Methyl-propargylamine (R-2HMP), a new potent monoamine oxidase inhibitor and antiapoptotic agent. Drug Metab Dispos. 28(2):147-54.
Fliss H, Gattinger D. 1996. Apoptosis in ischemic and reperfused rat myocardium. Circ Res 79:949-956.
Haunstetter A, Izumo S. 1998. Apoptosis: basic mechanisms and implications for cardiovascular disease. Circ Res 82:1111-1129.
Hershkowitz A, Wolfgram LJ, Rose NR et al. 1987. Coxsackievirus B3 murine myocarditis: a pathologic spectrum of myocarditis in genetically defined inbred strains. J Am Coll Cardiol 9:1311-1319.
Jeremias I, Kupatt C, Villalba-Martin et al. 2000. Involvement of CD95/Apo-1/Fas in cell death after myocardial ischemia. Circulation 102:915-920.
Tatton, et al., 1993. Selegiline Can Mediate Neuronal Rescue Rather than Neuronal Protection. Movement Disorders 8 (Supp. 1):S20-S30.
Tatton, et al. 1991. Rescue of Dying Neurons," J. Neurosci. Res. 30:666-672.
Yamaguchi s, Yamaoka M, Okuyama M et al. 1998. Elevated circulation levels and cardiac secretion of soluble Fas ligand in patients with congestive heart failure. Am J Cardiol 83:1500-1503.
Yaniv G, Shilkrut M, Lotan R, Larish S, Berke G, Binah O. 2002. "Hypoxia predisposes neonatal rat ventricular myocytes to apoptosis induced by activation of the Fas (CD95/Apo-1) receptor: Fas activation and apoptosis in hypoxic myocytes". Cardiovasc Res 54:611-623.
Yaoita H, Ogawa K, Macehara K, Maruyama Y. 1998. Attenuation of ischemia reperfusion injury in rats by a caspase inhibitor. Circulation 97:276-281.
Zimmermann K, Waldmeier PC, Tatton WG. 1999. Dibenzoxepines as treatments for neurodegenerative diseases. Pure Appl Chem 71(11):2039-2046.

## Claims

1. Use of S-(-)-N-propargyl-1-aminoindan or a pharmaceutically acceptable salt thereof for the preparation of a pharmaceutical composition for prevention and/or treatment of a cardiovascular disease or disorder selected from myocardial infarction, myocardial ischemia, or myocardial ischemia and reperfusion.

2. The use according to claim 1, for preventing and/or treating myocardial ischemia or myocardial ischemia and reperfusion.

3. The use according to claim 1 or 2, wherein a pharmaceutically acceptable salt of S-(-)-N-propargyl-1-aminoindan is used.

4. The use according to claim 3, wherein said pharmaceutically acceptable salt is selected from the group consisting of the mesylate salt, the maleate salt, the fumarate salt, the tartrate salt, the hydrochloride salt, the hydrobromide salt, the esylate salt, the p-toluenesulfonate salt, the benzoate salt, the acetate salt, the phosphate salt and the sulfate salt of S-(-)-N-propargyl- I -aminoindan.

5. A pharmaceutical composition comprising S-(-)-N-propargyl-1-aminoindan or a pharmaceutically acceptable salt thereof for use in the prevention and/or treatment of a cardiovascular disease or disorder selected from myocardial infarction, myocardial ischemia, or myocardial ischemia and reperfusion.

6. The pharmaceutical composition according to claim 5 for use in preventing and/or treating myocardial ischemia or myocardial ischemia and reperfusion.

7. The pharmaceutical composition for use according to claims 5 or 6 wherein a pharmaceutically acceptable salt of S-(-)-N-propargyl-1-aminoindan is used.

8. The pharmaceutical composition for use according to claim 7, wherein said pharmaceutically acceptable salt is selected from the group consisting of the mesylate salt, the maleate salt, the fumarate salt, the tartrate salt, the hydrochloride salt, the hydrobromide salt, the esylate salt, the p-toluenesulfonate salt, the benzoate salt, the acetate salt, the phosphate salt and the sulfate salt of S-(-)-N-propargyl-1-aminoindan.

## Patentansprüche

1. Verwendung von S-(-)-N-Propargyl-1-aminoindan oder eines pharmazeutisch verträglichen Salzes davon zur Herstellung einer pharmazeutischen Zusammensetzung zur Prävention und/oder Behandlung einer kardiovaskulären Erkrankung oder Störung, ausgewählt aus Myokardinfarkt, Myokardischämie, oder Myokardischämie und Reperfusion.

2. Die Verwendung gemäß Anspruch 1 zur Prävention und/oder Behandlung von Myokardischämie, oder Myokardischämie und Reperfusion.

3. Die Verwendung gemäß Anspruch 1 oder 2, wobei ein pharmazeutisch verträgliches Salz von S-(-)-N-Propargyl-1-aminoindan verwendet wird.

4. Die Verwendung gemäß Anspruch 3, wobei das pharmazeutisch verträgliche Salz ausgewählt ist aus der Gruppe, bestehend aus dem Mesylatsalz, dem Maleatsalz, dem Fumaratsalz, dem Tartratsalz, dem Hydrochloridsalz, dem Hydrobromidsalz, dem Esylatsalz, dem p-Toluolsulfonatsalz, dem Benzoatsalz, dem Acetatsalz, dem Phosphatsalz und dem Sulfatsalz von S-(-)-N-Propargyl-1-aminoindan.

5. Eine pharmazeutische Zusammensetzung umfassend S-(-)-N-Propargyl-1-aminoindan oder ein pharmazeutisch verträgliches Salz davon zur Verwendung in der Prävention und/oder Behandlung einer kardiovaskulären Erkrankung oder Störung, ausgewählt aus Myokardinfarkt, Myokardischämie, oder Myokardischämie und Reperfusion.

6. Die pharmazeutische Zusammensetzung gemäß Anspruch 5 zur Verwendung in der Prävention und/oder Behandlung von Myokardischämie, oder Myokardischämie und Reperfusion.

7. Die pharmazeutische Zusammensetzung zur Verwendung gemäß Anspruch 5 oder 6, wobei ein pharmazeutisch verträgliches Salz von S-(-)-N-Propargyl-1-aminoindan verwendet wird.

8. Die pharmazeutische Zusammensetzung zur Verwendung gemäß Anspruch 7, wobei das pharmazeutisch verträgliche Salz ausgewählt ist aus der Gruppe, bestehend aus dem Mesylatsalz, dem Maleatsalz, dem Fumaratsalz, dem Tartratsalz, dem Hydrochloridsalz, dem Hydrobromidsalz, dem Esylatsalz, dem p-Toluolsulfonatsalz, dem Benzoatsalz, dem Acetatsalz, dem Phosphatsalz und dem Sulfatsalz von S-(-)-N-Propargyl-1-aminoindan.

## Revendications

1. Utilisation d'un S-(-)-N-propargyl-1-aminoindane ou d'un sel pharmaceutiquement acceptable de celui-ci pour la préparation d'une composition pharmaceutique pour la prévention et/ou le traitement d'une maladie ou d'un trouble cardiovasculaire choisi parmi l'infarctus du myocarde, l'ischémie myocardique, ou l'ischémie-reperfusion myocardiques.

2. Utilisation selon la revendication 1 pour la prévention et/ou le traitement de l'ischémie myocardique ou de l'ischémie-reperfusion myocardiques.

3. Utilisation selon la revendication 1 ou 2, dans laquelle un sel pharmaceutiquement acceptable du S-(-)-N-propargyl-1-aminoindane est utilisé.

4. Utilisation selon la revendication 3, dans laquelle ledit sel pharmaceutiquement acceptable est choisi dans le groupe constitué par le sel de mésylate, le sel de maléate, le sel de fumarate, le sel de tartrate, le sel chlorhydrate, le sel de bromhydrate, le sel d'ésylate, le sel de p-toluènesulfonate, le sel de benzoate, le sel d'acétate, le sel de phosphate et le sel de sulfate du S-(-)-N-propargyl-1-aminoindane.

5. Composition pharmaceutique comprenant un S-(-)-N-propargyl-1-aminoindane ou un sel pharmaceutiquement acceptable de celui-ci pour son utilisation dans la prévention et/ou le traitement d'une maladie ou d'un trouble cardiovasculaire choisi parmi l'infarctus du myocarde, l'ischémie myocardique, ou l'ischémie-reperfusion myocardiques.

6. Composition pharmaceutique selon la revendication 5 pour son utilisation dans la prévention et/ou le traitement de l'ischémie myocardique ou de l'ischémie-reperfusion myocardiques.

7. Composition pharmaceutique pour son utilisation selon les revendications 5 ou 6 dans laquelle un sel pharmaceutiquement acceptable du S-(-)-N-propargyl-1-aminoindane est utilisé.

8. Composition pharmaceutique pour son utilisation selon la revendication 7, dans laquelle ledit sel pharmaceutiquement acceptable est choisi dans le groupe constitué par le sel de mésylate, le sel de maléate, le sel de fumarate, le sel de tartrate, le sel chlorhydrate, le sel de bromhydrate, le sel d'ésylate, le sel de p-toluènesulfonate, le sel de benzoate, le sel d'acétate, le sel de phosphate et le sel de sulfate du S-(-)-N-propargyl-1-aminoindane.
